# EUROPEAN PATENT SPECIFICATION

(11) **EP 1 529 445 B1**
(45) Date of publication and mention of the grant of the patent: **21.02.2007**
(21) Application number: 03388070.9
(22) Date of filing: 04.11.2003
(51) Int. Cl.: A23L 1/30, A61K 36/00, A61K 36/9068, A61K 36/42, A61K 36/484, A61K 36/67

(54) **Dietary supplement composition**
Zubereitung für ein Nährungsergänzungsmittel
Composition pour supplément nutritionnel

(43) Date of publication of application: 11.05.2005
(73) Proprietor: Korfitsen, Jens Peter Corfitz, 5444 Künten (CH); Indian Herbs, 1030 Wien, Arsenal, Objekt 3/1/8 (AT)
(72) Inventor: Gupte, Shireesh S., A-1030 Wien, Arsenal (AT)
(74) Representative: Rotne, Jens Styrup

(56) References cited:
- WO-A-01/03716
- WO-A-95/08318
- RU-C- 2 160 533
- US-A1- 2002 122 834
- US-B1- 6 586 018
- DATABASE BIOSIS [Online] BIOSCIENCES INFORMATION SERVICE, PHILADELPHIA, PA, US; 1982 GARG SK: "Anti fertility effect of Embelia-ribes and Piper-longum in female rats" Database accession no. 198375020883 XP002274807

## Description

The present invention relates to a dietary supplement composition comprising a vegetable protein containing carrier, a vegetable source for sugar, a vegetable source for fat, salt and up to about 6 weight-% of an active ingredient composed of five different plants as constituents.

Dietary supplement compositions based on plants are well-known. Such dietary supplement compositions are widely used for a large variety of applications, and mainly applications for reasons of health. Thus, several dietary supplement compositions have proven to improve health in humans and animals, e.g. in respect of immune response, heart conditions, general conditions etc. Of course the dietary supplement composition for a specific use must be specifically designed for this use and fulfil the requirement set by the use. Although the function of one single plant on an animal organism may be well-known and documented, the function of several plants in combination is difficult to foresee, as interference between the plant properties may lead to unpredictable effects, some of which may be desirable, others highly undesirable. Consequently, it is very often a difficult task to design dietary supplement compositions based on plants for specific purposes. This is a task which require detailed knowledge of plants and their behaviour in animal organisms and careful studies. When a dietary supplement composition has been successfully formulated, it is usually well-functioning, environmental friendly and with none or negligible side effects compared to artificial chemical compositions for the same application. Despite the fact that numerous dietary supplements based on plants for numerous applications are commercially availably, only a few relates to dietary supplement compositions that can improve the fertility in mammals. Compositions based on plants for improving fertility in animals are known from e.g. the disclosures of WO 99/66877, WO 95/08318, and US 2002/122834 A1. There is, however, still a need for an effective dietary supplement composition that can improve the fertility in mammals, and in particular farm animals.

An object of the present invention is to provide a dietary supplement composition based on plants that can improve the fertility in mammals.

This object is achieved by the invention as defined in the claims. ,

According to the invention a dietary supplement composition is provided that has proven to demonstrate excellent properties in respect of improving fertility health in mammals and in particular the fertility health in farm animals like pigs, horses and cattle. Moreover, as a secondary factor, the dietary supplement composition according to the invention has proven to possess unexpected good qualities of improving the resistance in animals towards diseases caused by bacteria and virus. Consequently, the invention provides a dietary supplement composition capable of improving the general health, and in particular improving the fertility rate in mammals, without any adverse side-effects. The dietary supplement composition according to the invention can, therefore, be used as an alternative to prostaglandines and other similar substances, which in terms of general health and environment is undesired.

In a first aspect, the present invention re-lates to a dietary supplement composition comprising 50-70 weight-% of a carrier that contains vegetable protein, 15-25 weight-% of a vegetable source for sugar, 3-15 weight-% of a vegetable source for fat, 7-14 weight-% salt and 1-6 weight-% of an active ingredient, where the aforementioned weight percentages are based on the total weight of the dietary supplement composition, and the active ingredient, with weight percentages based on the total weight of the active ingredient, is constituted by a mixture of:
14-26 weight-% of zingiber offizinalis,
14-26 weight-% of piper longum,
14-26 weight-% of piper nigrum,
14-26 weight-% of cucumis trigonus, and
14-26 weight-% of glycyrrhiza glabra.

The active ingredient with the balanced amounts of the five plants has proven to provide excellent properties to the dietary supplement composition according to the invention. The amount of plants to be used in the active ingredient depends on the quality of the harvest of the individual plants. Depending on the analysis of the plants the five plants may present in substantially equal amounts like 19-21 weight-% of each plants, more likely 17-23 weight-% of each plant and normally in the range of 14-26 weight-% of each plant. The harvested plants are analysed for water content, protein content and other organic substances, and from the analysis the skilled person is able to decide the amount of the individual plants in the active ingredient as a matter of routine.

Although the carrier may be any cereal product that contains protein in an amount of about 8-10 weight-% or more, it is, however, preferred that the carrier is oat, barley or maize, or optionally mixtures of two or all of these. More preferred the carrier is oat, the properties of which have proven to be good in relation to the dietary supplement composition according to the invention.

The source for sugar in the dietary supplement composition according to invention may, in principle be any plant or fruit that contains a sufficient amount of sugar, e.g. about 30-40 weight-% or more, but the preferred source for sugar is carob, sugar beet, or sugar cane. More preferred the source for sugar is carob. Normally, carob can comprise up to about 50 weight-% vegetable sugar, making the sugar content of carob ideal for the dietary supplement composition according to the invention.

The source for vegetable fat is conveniently selected from nuts, oil seed rape, or seeds from sunflowers, which are all known to contain vegetable fats in rather large amounts, and preferably the source for vegetable fat are nuts, like hazel nuts, walnuts, pecans, brazil nuts and other nuts with high contents of fat. More preferred the source for fat is hazel nuts. The seed from the hazel nut usually contains fatty oil in an amount of about 50-80 weight-%. It is convenient that the source for vegetable fat comprises 45-55 weight-% fat or more.

The dietary supplement composition according to the invention comprises salt in an amount of 7-14 weight-%, preferably 8-12 weight-%, and preferably the salt is substantially NaCl. "Substantially NaCl₂" is to be understood in such a way that the salt for the major part is NaCl which may comprises other constituents like potassium chloride, calcium and magnesium salts and impurities normally present in common commercially available salt. The relative large amount of salt serves to help initiating the hormone production with regard to oestrus, ovulation etc.

Furthermore, in order to improve the dietary supplement composition according to the invention, the composition may additionally comprise one or more metal complexes in an amount of 0.04-0.6 weight-% based on the total weight of the dietary supplement composition. Preferably, the one or more metal complexes are selected among zinc complexes, iron complexes, magnesium complexes and selenium complexes, which are normally referred to as micro elements. More preferred the metal complex is a selenium complex. Selenium affects the hormonal production in the liver and thereby helps to improve the fertility effect of the dietary supplement according to the invention.

Likewise, in order to improve the dietary supplement, the composition may further comprise one or more vitamins in an amount of 0.5-1.8 weight-% based on the total weight of the composition. Preferably the vitamins are one or more of vitamin A, vitamin C, vitamin B₆, vitamin B₁₂, vitamin D₃, and vitamin E. More preferred the vitamins are vitamin A, vitamin C and vitamin D₃ in combination.

The dietary supplement composition according to the invention may further comprise flavour and/or sweetener in an amount of 0.1-1.5 weight-% based on the total weight of the composition. When both flavour and sweetener are present, the flavour preferably constitutes 0.25-1.25 weight-% based on the total weight of the composition, and the sweetener preferably constitutes 0.05-0.75 weight-% based on the total weight of the composition. The flavour may be any commercially available flavour that is suitable for use in the composition and is attractive for the target animal. Natural flavours are preferred. The sweetener may also be any commercially available sweetener suitable for use in the composition. Natural sweeteners are preferred. The main purpose of the flavour and sweetener is to make the taste of the dietary supplement composition according to the invention more attractive for species intended to eat the composition.

According to the invention the dietary supplement composition comprises the active ingredient in an amount of 1-6 weight-%, and preferably in an amount of 2-4 weight-% based on the total weight of the dietary supplement composition. The plants of the active ingredient interact to provide a synergetic effect. Moreover, it is also believed that the active ingredient interacts with the other constituents of the dietary supplement composition to develop the excellent properties of the dietary supplement composition according to the invention. The active ingredient and the function of the constituents of the active ingredient will be described in details in subsequent paragraphs.

In a second aspect, the invention relates to a method for producing a dietary supplement composition. The method comprises the step of mixing the following constituents based on the total weight of the composition:
about 50-70 weight-% of a vegetable protein containing carrier,
about 15-25 weight-% of a vegetable source for sugar,
about 3-15 weight-% of a vegetable source for fat,
about 7-14 weight-% salt, and
about 1-6 weight-% of an active ingredient
to form a substantially homogeneous mixture, wherein the active ingredient is added as a substantially homogeneous premix constituting of the following, based on the total weight of said active ingredient:
14-26 weight-% of zingiber offizinalis,
14-26 weight-% of piper longum,
14-26 weight-% of piper nigrum,
14-26 weight-% of cucumis trigonus, and
14-26 weight-% of glycyrrhiza glabra.

Preferably, the vegetable protein containing carrier is selected from oat, barley or maize; the vegetable protein containing carrier is preferably selected from oat.

In a preferred embodiment of the method according to the invention the vegetable source for sugar is selected from carob, sugar beet, or sugar cane, the vegetable source for sugar is preferably selected from carob.

Although the vegetable source for fat may be selected from any plants having a high content of vegetable fat; according to the method it is, however, preferred that the vegetable source for fat is selected from nuts, sunflower seeds, oil seed rape, more preferred the vegetable source for fat is selected from nuts, and even more preferred the vegetable source for fat is selected from hazel nuts.

Moreover, it is preferred that the composition is admixed with sweetener and/or flavour in an amount of about 0.1-1.5 weight-% based on the total weight of the composition to improve the taste.

In a preferred embodiment of the method according to invention the method comprises the further step of pelletizing the produced dietary supplement composition into pellets, preferably into pellets with diameters in the range of 2-18 mm.

The dietary supplement is preferably given in amounts of 40 to 500 g per day for 2 to 10 days, preferably 60 to 200 g per day for 3 to 10 days, more preferred 80 to 165 g for 3 to 7 days. The dietary supplement is preferably given in the metoestrus period, but may also be given in the dioestrus or prooestus period.

In a third aspect, the invention relates to use of a dietary supplementary composition as described above for improving the fertility in mammals. Preferably, the mammals are farm animals, more preferred pigs, horses and cattle.

Furthermore, the invention also relates to the use of the dietary supplement composition as previously described for the manufacture of a medical or dietary composition for improving resistance to illness caused by bacteria or virus in mammals.

The invention will now be described in further details with reference to an example and a drawing in which:
- Figure 1: shows the improvement in fertility for pigs given the dietary supplement composition according to the invention compared with a control group for trials ran in Austria.
- Figure 2: shows the improvement in fertility for dairy cows given the dietary supplement composition according to the invention compared with a control group.
- Figure 3: shows the improvement in fertility for pigs given the dietary supplement composition according to the invention compared with a control group For trials ran in Denmark.

The term "vegetable" as used herein is to be understood in the broadest sense, as including any known plant material, species of plants, parts from plants, including vegetables, legumes, fruits, herbs, nuts, flowers etc.

According to the invention the active ingredient is constituted by a mixture of:
14-26 weight-% of zingiber offizinalis,
14-26 weight-% of piper longum,
14-26 weight-% of piper nigrum,
14-26 weight-% of cucumis trigonus, and
14-26 weight-% of glycyrrhiza glabra.

Zingiber offizinalis, commonly known as ginger, comprises aromatic oils, terpene, d-camphene, β-phellandrene, sesquiterpene and zingerone. Zingiberoffizinalis has an effect towards thrombosis in blood vessels and improves the transportation of blood in the vessels. Moreover, zingiber offizinalis is an aphrodisiac.

Piper longum and piper nigrum (long pepper and pepper plant) belongs to the pepper family and comprise piperine, piperidine, chevacine and oils. They have an effect on urogenital diseases and also function as aphrodisiac.

Cucumis trigonus of the gourd family comprises polysacharide, glycoside, phytosterols, saponine and hydrocarbons. Cucumis trigonus can be used in case of ureter problems. Furthermore, it is cyklus stimulating and uterus active.

Glycyrrhiza glabra or liquorice comprises glycerrhizine and asparagine. Besides a rejuvenating effect it also has an ovary stimulating effect.

The present inventor has found that the mixture of the above plants in predetermined proportions has an unexpected good effect on the fertility of mammals. The dietary supplement according to the invention improves the current of blood and the mineral absorption in the uterus and ovary, stimulates the hormone production and facilitate the initiation of the sexual cyclus, thereby leading to a very high success of oestrus and pregnancy. The dietary supplement furthermore improves the resistance towards parasitic, bacterial and viral attacks. It has been found that the mutual proportions of the content of plants in the active ingredient should be about 14-26 weight-%. Consequently, an active ingredient with excellent qualities can be obtained when the five plants are present in about equal proportions. The mutual proportion of the individual plants in the active ingredient is determined by the skilled person on basis of analysis for water content, protein content and other organic substances. A synergetic effect has surprisingly been recognized as the result of the balanced mixture of the five plants in the active ingredient.

The active ingredient can be manufactured by mixing the finely divided plants in substantially dry state in the desired proportions, using procedures well-known to the persons skilled in the art.

The premixed active ingredient can be mixed immediately with the other constituents of the dietary supplement composition according to the invention, or the premixed active ingredient can be stored for later mixing.

According to a non-binding theory the active ingredient together with the vegetable protein, vegetable sugar and vegetable fat forms a unique mixture with improved properties in respect of improving the fertility and resistance to parasites and bacterial and viral attacks. It is believed that this combination is the reason for the excellent performance of the dietary supplement composition according to the invention, and that the full performance of the dietary supplement composition is achieved by the balanced interaction between the active ingredient and the vegetable protein, vegetable sugar, vegetable fat and salt. The dietary supplement may be further improved by addition of vitamins and micro elements, which are easier absorbed into the organs, due to the effect of the constituents.

### Example

### 1. Preparation of dietary supplement composition.

A dietary supplement composition according to the invention was prepared with the composition as given in table 1.

**Table 1. Dietary supplement composition**

| Material | Amount (weight-%) |
|---|---|
| Oat | 59.30 |
| Carob flour | 20.00 |
| Salt, NaCl₂ | 10.00 |
| Hazel nut flour | 5.00 |
| Active ingredient | 4.00 |
| Vitamins A,C,D₃ | 1.00 |
| Flavour | 0.50 |
| Sweetener | 0.10 |
| Selenium complex | 0.10 |

The oat, the carob flour, the salt, the hazel nut flour were mixed and the vitamins were added as a natural preparation containing vitamin A, C and D₃, The added flavour was a natural flavour of extracts from apples. The sweetener was fruit sugar and the selenium complex was a commercially available standard selenium complex. All the ingredients were commercially available grades, quality tested and containing no toxins.

The plants for the active ingredient was provided by Indian Herbs, Austria. The plants for the active ingredient were analysed for water content, protein content and other organic substances resulting in that the active ingredient was prepared as a premix with the composition given in table 2.

**Table 2. Active ingredient composition**

| Plant | Amount (weight-%) |
|---|---|
| Zingiber offizinalis | 20.8 |
| Piper longum | 23.2 |
| Piper nigrum | 19.1 |
| Cucumis Trigonus | 15.8 |
| Glycyrrhiza glabra | 21.1 |

The oat, carob, salt, hazel nut, vitamins, flavour, sweetener and selenium complex in powder form were mixed in an amount equal to 100 kg dietary supplement composition (96 kg) in a mixer. After a homogeneous mixture was obtained, 4 kg of the premix of active ingredient was added and mixed until homogeneity was obtained.

The mixture was then fed to a pelletizer and formed into pellets with an average size of about 2-5 mm, which were directly useable as dietary supplement composition.

### 2. Experimental data from sows and dairy cows.

### Trial 1.

Veterinary-University Vienna, Austria.

The dietary supplement composition prepared as described above was tested in sows as outlined below:
During the period 2001-2002 Dr. Maximillian Dobrets-berger supervised a trial where the dietary supplement according to the invention was given to a group of sows (165 g per day per sow for 3 days, total approx 500 g per sow) and compared with a control (0-group). During the trial period all data from each litter was registered. During the period there was a total of 176 litters. In the table below given the average results from both groups.

**Table 1: results per litter**

| Parameter | Dietary supplement | Control |
|---|---|---|
| Total number of piglets | 12.08 | 11.12 |
| Living born piglets | 10.94 | 9.8 |
| Living born from porkers | 10.24 | 9.32 |
| Living born from sows | 11.28 | 9.94 |
| "Days" till next pregnancy | 17.71 | 27.21 |

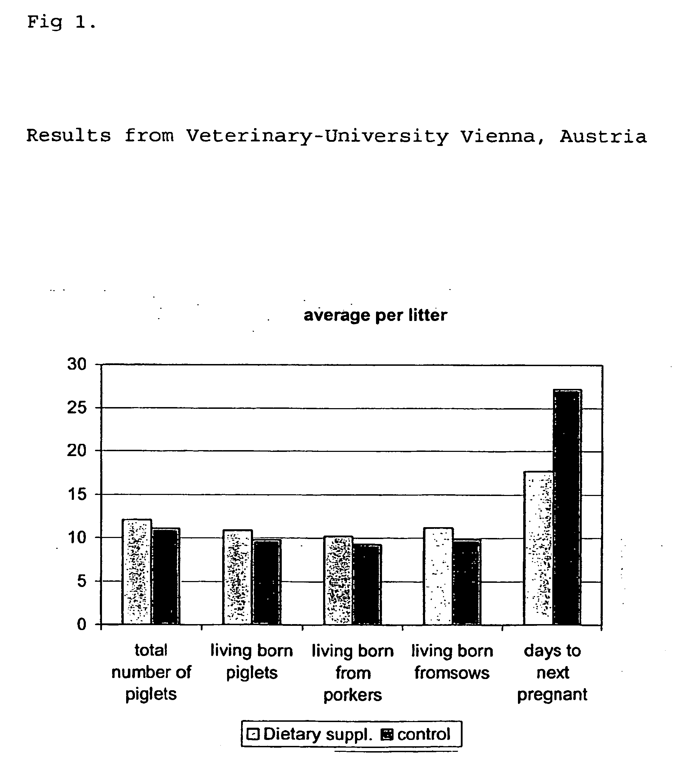

The results are also depicted graphically in fig. 1. It is evident that there is a significant difference between the group receiving the dietary supplement according to the invention and the control group.
In all the parameters in the test, the group receiving the dietary supplement according to the invention obtained significant better results than the control group.

### Trial 2.

The dietary supplement according to the invention was tested on 80 dairy cows in Austria.

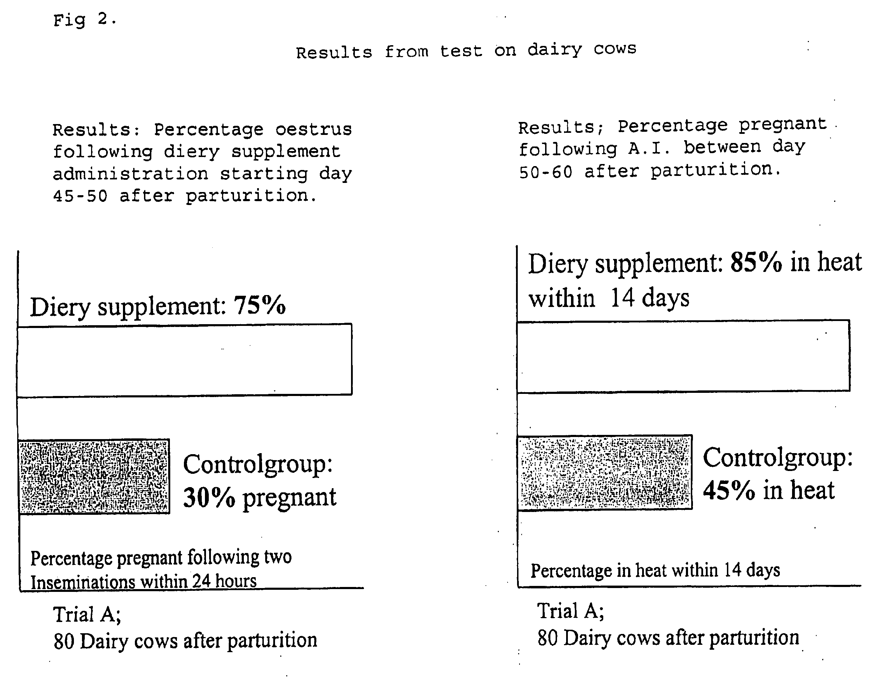

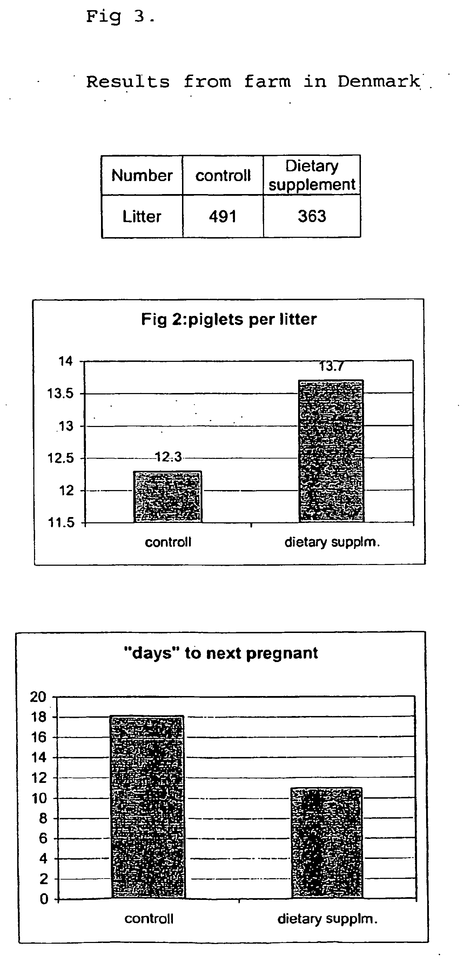

Figure 2 shows the result of tests on dairy cows with the dietary supplement composition according to the invention. The tests were carried out on a-cyclic dairy cows. The administration of the dietary supplement composition were 330 g/per animal for 3 days, or 200 g /per animal for 5 days, making a total of about 1000 g/per animal. The administration took place 10 to 14 days after absent or recent oestrous.
It is clearly seen that the group receiving the dietary supplement according to the invention has significantly improved fertility data compared to the control group.

### Trial 3.

Use of a dietary supplement composition according to the invention in sows to achieve better post weaning results.
Time: October/November 2001
Place: Austria
Test animals: 1236 Large white pig on one site; average age 2,8 years; average number of litter before trial 3,7
Insemination: A.I. (artificial insemination) with diluted semen; 2x 24 and 36 hours after onset of oestrous
Dietary supplement composition administration: 5 days; 20g each day; total amount 100g - beginning with the day of weaning until day 4 (day 0-4 post-weaning)
Oestrous detection: With boar
Pregnancy detection: With ultrasonics on day 30 post insemination and with the boar on day 21 after insemination (absence of oestrous)
Feeding: Standard post weaning feed and vitamin and mineral flush on day 3 after weaning

### (Standard procedure on farm since 2 years)

Comparison: 2 latest results of periods before use of the dietary supplement composition according to the invention.

| Group | No. of pigs | No. insem. | No. pregnant | % insem / pregnant |
|---|---|---|---|---|
| Comp. Group I | 1168 | 1142 | 1004 | 98/86 |
| Comp. Group II | 1080 | 1002 | 912 | 93/84 |
| Trial Group Dietary suppl. composition | 1264 | 1264 | 1262 | 100/99 |

The dietary supplement composition according to the invention based on natural ingredients was tested as an oestrous inducer for post weaning oestrous in pig on a large commercial farm in Austria. The product was examined for the inclusion of hormones, antibiotics and other undesirable substances; e.g. heavy metals, pesticides. No forbidden or undesirable substances were found. The results of the trial show a significant raise in oestrous appearance and following pregnancy compared to results achieved in the periods before the use of the dietary supplement composition. This is in accordance with previously seen effects in sows in university and field trials from Italy, Austria and Spain. The overall results were very impressing especially when considering that the results of the farm were already good.

### Trial 4.

The dietary supplement according to the invention was tested on sows on a farm in Denmark in 2003. The number of litters per sow and size of litters were recorded.
The number of sows was 440. The group tested with the dietary supplement according to the invention was given 100 g per day per sow for 5 days, and total approx. 500 g per sow. The number of litter per sow per year before the trial was 2,4.
The results of the trial are shown in figure 3. It is clearly seen that the sows given the dietary supple- , ment had larger litters and fewer days between pregnancy. Thus, the sows given the dietary supplement were significant more productive.

The dietary supplement according to the invention improves rates of pregnancy after insemination, reduces the numbers of days between pregnancies and for sows the numbers of piglets per litter is increased. Consequently, the invention provides a dietary supplement composition that improves the fertility health in farm animals and, thereby, also contributes to improve the economic turnover.

## Claims

1. A dietary supplement composition comprising 50-70 weight-% of a vegetable protein containing carrier, 15-25 weight-% of a vegetable source for sugar, 3-15 weight-% of a vegetable source for fat, 7-14 weight-% salt and 1-6 weight-% of active ingredient based on the total weight of the dietary supplement composition, wherein the active ingredient based on the total weight of said active ingredient is constituted by a mixture of:
14-26 weight-% of zingiber offizinalis,
14-26 weight-% of piper longum,
14-26 weight-% of piper nigrum,
14-26 weight-% of cucumis trigonus, and
14-26 weight-% of glycyrrhiza glabra.

2. A dietary supplement composition according to claim 1, wherein the protein containing carrier is selected from oat, barley or maize, preferably oat.

3. A dietary supplement composition according to claim 1, wherein the source for sugar is selected from carob, sugar beet, or sugar cane, preferably the source for sugar is carob.

4. A dietary supplement composition according to claim 1, wherein the source for fat is selected from nuts, oil seed rape, or seeds from sunflowers, preferably the source for fat are nuts, more preferred the source for fat are hazel nuts.

5. A dietary supplement composition according to claim 1, wherein the salt is substantially NaCl.

6. A dietary supplement composition according to claim 1, wherein the composition further comprises one or more metal complexes in an amount of 0.04-0.6 weight-% based on the total weight of the dietary supplement composition.

7. A dietary supplement composition according to claim 6, wherein the one or more metal complexes are selected among zinc complexes, iron complexes, magnesium complexes and selenium complexes, preferably the metal complex is a selenium complex.

8. A dietary supplement composition according to claim 1, wherein the composition further comprises one or more vitamins in an amount of 0.5-1.8 weight-% based on the total weight of the composition.

9. A dietary supplement composition according to claim 7, wherein the composition comprises one or more of the vitamins: vitamin A, vitamin C, vitamin B₆, vitamin B₁₂, vitamin D₃, and vitamin E.

10. A dietary supplement composition according to claim 1, wherein the composition further comprises flavour and/or sweetener in an amount of 0.1-1.5 weight-% based on the total weight of the composition.

11. A dietary supplement composition according to claim 1, wherein the active ingredient is present in an amount of 2-4 weight-% based on the total weight of the dietary supplement composition.

12. A method for producing a dietary supplement composition comprising the step of mixing the following constituent based on the total weight of the composition:
about 50-70 weight-% of a vegetable protein containing carrier,
about 15-25 weight-% of a vegetable source for sugar,
about 3-15 weight-% of a vegetable source for fat,
about 7-14 weight-% salt, and
about 1 to 6 weight-% of an active ingredient
to form a substantially homogeneous mixture, wherein the active ingredient is added as a substantially homogeneous premix constituted of the following, based on the total weight of said active ingredient:
14-26 weight-% of zingiber offizinalis,
14-26 weight-% of piper longum,
14-26 weight-% of piper nigrum,
14-26 weight-% of cucumis trigonus, and
14-26 weight-% of glycyrrhiza glabra.

13. A method according to claim 12, wherein the vegetable protein containing carrier is selected from oat, barley or maize, preferably the vegetable protein containing carrier is selected from oat.

14. A method according to claim 12 or 13, wherein the vegetable source for sugar is selected from carob, sugar beet, or sugar cane, preferably the vegetable source for sugar is selected from carob.

15. A method according to claim 12, 13 or 14, wherein the vegetable source for fat is selected from nuts, sunflower seeds, oil seed rape, preferably the vegetable source for fat is selected from nuts, more preferred the vegetable source for fat is selected from hazel nuts.

16. A method according to claims 12-15, wherein the composition for mixing further comprises sweetener and/or flavour in an amount of about 0.1-1.5 weight-% based on the total weight of the composition.

17. A method according to any of the claims claim 12-16 comprising the further step of pelletizing the produced dietary supplement composition into pellets, preferably into pellets with diameters in the range of 5-25 mm.

18. Use of the dietary supplement composition according to claims 1-11 for improving the fertility in mammals.

19. Use according to claim 18, wherein the mammals are farm animals, preferably pigs, horses and cattle.

20. Use of the dietary supplement composition according to claims 1-11 for the manufacture of a medical or dietary composition for improving resistance to illness caused by bacteria or virus in mammals.

## Patentansprüche

1. Nahrungsergänzungszusammensetzung, umfassend 50 bis 70 Gew.-% eines ein pflanzliches Protein enthaltenden Trägers, 15 bis 25 Gew.-% einer pflanzlichen Zuckerquelle, 3 bis 15 Gew.-% einer pflanzlichen Fettquelle, 7 bis 14 Gew.-% Salz und 1 bis 6 Gew.-% eines Wirkstoffs, bezogen auf das Gesamtgewicht der Nahrungsergänzungszusammensetzung, wobei der Wirkstoff, bezogen auf das Gesamtgewicht des Wirkstoffs, aus einem Gemisch aus
14 bis 26 Gew.-% *Zingiber officinalis*
14 bis 26 Gew.-% *Piper longum*
14 bis 26 Gew.-% *Piper nigrum*
14 bis 26 Gew.-% *Cucumis trigonus und*
14 bis 26 Gew.-% *Glycyrrhiza glabra*
besteht.

2. Nahrungsergänzungszusammensetzung nach Anspruch 1, in der der proteinhaltige Träger aus Hafer, Gerste oder Mais, vorzugsweise Hafer ausgewählt ist.

3. Nahrungsergänzungszusammensetzung nach Anspruch 1, in der die Zuckerquelle aus Johannisbrot, Zuckerrübe oder Zuckerrohr ausgewählt ist; vorzugsweise ist die Zuckerquelle Johannisbrot.

4. Nahrungsergänzungszusammensetzung nach Anspruch 1, in der die Fettquelle aus Nüssen, Ölsaatraps oder Sonnenblumenkörnern ausgewählt ist; vorzugsweise sind Nüsse die Fettquelle; stärker bevorzugt sind Haselnüsse die Fettquelle.

5. Nahrungsergänzungszusammensetzung nach Anspruch 1, in der das Salz im Wesentlichen NaCl ist.

6. Nahrungsergänzungszusammensetzung nach Anspruch 1, wobei die Zusammensetzung außerdem einen oder mehrere Metallkomplexe in einer Menge von 0,04 bis 0,6 Gew.-%, bezogen auf das Gesamtgewicht der Nahrungsergänzungszusammensetzung, enthält.

7. Nahrungsergänzungszusammensetzung nach Anspruch 6, wobei der eine oder die mehreren Metallkomplexe aus Zinkkomplexen, Eisenkomplexen, Magnesiumkomplexen und Seleniumkomplexen ausgewählt sind; vorzugsweise ist der Metallkomplex ein Selenkomplex.

8. Nahrungsergänzungszusammensetzung nach Anspruch 1, wobei die Zusammensetzung außerdem ein oder mehrere Vitamine in einer Menge von 0,5 bis 1,8 Gew.-%, bezogen auf das Gesamtgewicht der Zusammensetzung, enthält.

9. Nahrungsergänzungszusammensetzung nach Anspruch 7, wobei die Zusammensetzung eines oder mehrere der Vitamine Vitamin A, Vitamin C, Vitamin B₆, Vitamin B₁₂, Vitamin D₃ und Vitamin E umfasst.

10. Nahrungsergänzungszusammensetzung nach Anspruch 1, wobei die Zusammensetzung außerdem Geschmacks- und/oder Süßstoff in einer Menge von 0,1 bis 1,5 Gew.-%, bezogen auf das Gesamtgewicht der Zusammensetzung, enthält.

11. Nahrungsergänzungszusammensetzung nach Anspruch 1, wobei der Wirkstoff in einer Menge von 2 bis 4 Gew.-%, bezogen auf das Gesamtgewicht der Nahrungsergänzungszusammensetzung, vorliegt.

12. Verfahren zur Herstellung einer Nahrungsergänzungszusammensetzung, umfassend den Schritt des Mischens der folgenden Bestandteile, bezogen auf das Gesamtgewicht der Zusammensetzung:
etwa 50 bis 70 Gew.-% eines ein pflanzliches Protein enthaltenden Trägers,
etwa 15 bis 25 Gew.-% einer pflanzlichen Zuckerquelle,
etwa 3 bis 15 Gew.-% einer pflanzlichen Fettquelle,
etwa 7 bis 14 Gew.-% Salz und
etwa 1 bis 6 Gew.-% eines Wirkstoffs,
um ein im Wesentlichen homogenes Gemisch herzustellen, in dem der Wirkstoff als im Wesentlichen homogene Vormischung, die, bezogen auf das Gesamtgewicht des Wirkstoffs, aus folgenden besteht:
14 bis 26 Gew.-% *Zingiber officinalis*
14 bis 26 Gew.-% *Piper longum*
14 bis 26 Gew.-% *Piper nigrum*
14 bis 26 Gew.-% *Cucumis trigonus und*
14 bis 26 Gew.-% *Glycyrrhiza glabra*
zugesetzt wird.

13. Verfahren nach Anspruch 12, bei dem der das pflanzliche Protein enthaltende Träger aus Hafer, Gerste oder Mais ausgewählt wird; vorzugsweise wird der das pflanzliche Protein enthaltende Träger aus Hafer ausgewählt.

14. Verfahren nach Anspruch 12 oder 13, bei dem die pflanzliche Zuckerquelle aus Johannisbrot, Zuckerrüben oder Zuckerrohr ausgewählt wird; vorzugsweise wird die pflanzliche Zuckerquelle aus Johannisbrot ausgewählt.

15. Verfahren nach Anspruch 12, 13 oder 14, bei dem die pflanzliche Fettquelle aus Nüssen, Sonnenblumenkernen, Ölsaatraps ausgewählt wird; vorzugsweise wird die pflanzliche Fettquelle aus Nüssen ausgewählt; stärker bevorzugt wird die pflanzliche Fettquelle aus Haselnüssen ausgewählt.

16. Verfahren nach Anspruch 12 bis 15, bei dem die Zusammensetzung zum Mischen außerdem Süß- und/oder Geschmacksstoff in einer Menge von etwa 0,1 bis 1,5 Gew.-%, bezogen auf das Gesamtgewicht der Zusammensetzung, enthält.

17. Verfahren nach einem der Ansprüche 12 bis 16, das den zusätzlichen Schritt der Pelletisierung der hergestellten Nahrungsergänzungszusammensetzung zu Pellets, vorzugsweise zu Pellets mit Durchmessern im Bereich von 5 bis 25 mm, umfasst.

18. Verwendung der Nahrungsergänzungszusammensetzung nach Anspruch 1 bis 11 zur Verbesserung der Fertilität bei Säugern.

19. Verwendung nach Anspruch 18, wobei die Säuger Nutztiere, vorzugsweise Schweine, Pferde und Rinder sind.

20. Verwendung der Nahrungsergänzungszusammensetzung nach Anspruch 1 bis 11 zur Herstellung einer Arzneimittel- oder Nahrungsergänzungszusammensetzung zur Verbesserung der Widerstandsfähigkeit gegen durch Bakterien oder Viren verursachte Krankheiten bei Säugern.

## Revendications

1. Composition pour complément alimentaire comprenant 50 à 70 % en poids d'un véhicule contenant une protéine végétale, 15 à 25 % en poids d'une source végétale de glucide, 3 à 15 % en poids d'une source végétale de lipide, 7 à 14 % en poids de sel et 1 à 6 % en poids de principe actif par rapport au poids total de la composition pour complément alimentaire, dans laquelle le principe actif, basé sur le poids total dudit principe actif, est constitué par un mélange de :
- 14 à 26 % en poids de *zingiber offizinalis,*
- 14 à 26 % en poids de *piper longum,*
- 14 à 26 % en poids de *piper nigrum,*
- 14 à 26 % en poids de *cucumis trigonus,* et
- 14 à 26 % en poids de *glycyrrhiza glabra.*

2. Composition pour complément alimentaire selon la revendication 1, dans laquelle le véhicule contenant la protéine est choisi parmi l'avoine, l'orge ou le maïs, de préférence l'avoine.

3. Composition pour complément alimentaire selon la revendication 1, dans laquelle la source de glucide est choisie parmi la caroube, la betterave sucrière ou la canne à sucre, de préférence la source de glucide est la caroube.

4. Composition pour complément alimentaire selon la revendication 1, dans laquelle la source de lipide est choisie parmi les noix, l'huile de colza ou les graines de tournesol, de préférence la source de lipide est choisie parmi les noix, de manière plus particulièrement préférée, la source de lipide est choisie parmi les noisettes.

5. Composition pour complément alimentaire selon la revendication 1, dans laquelle le sel est essentiellement NaCl.

6. Composition pour complément alimentaire selon la revendication 1, dans laquelle la composition comprend en outre un ou plusieurs complexes métalliques à raison de 0,04 à 0,6 % en poids par rapport au poids total de la composition pour complément alimentaire.

7. Composition pour complément alimentaire selon la revendication 6, dans laquelle un ou plusieurs de ces complexes métalliques sont choisis parmi les complexes du zinc, les complexes du fer, les complexes du magnésium et les complexes du sélénium, de préférence le complexe métallique est un complexe du sélénium.

8. Composition pour complément alimentaire selon la revendication 1, dans laquelle la composition comprend en outre une ou plusieurs vitamines à raison de 0,5 à 1,8 % en poids par rapport au poids total de la composition.

9. Composition pour complément alimentaire selon la revendication 7, dans laquelle la composition comprend une ou plusieurs vitamines : la vitamine A, la vitamine C, la vitamine B₆, la vitamine B₁₂, la vitamine D₃ et la vitamine E.

10. Composition pour complément alimentaire selon la revendication 1, dans laquelle la composition comprend en outre un arôme et/ou un édulcorant à raison de 0,1 à 1,5 % en poids par rapport au poids total de la composition.

11. Composition pour complément alimentaire selon la revendication 1, dans laquelle le principe actif est présent à raison de 2 à 4 % en poids par rapport au poids total de la composition pour complément alimentaire.

12. Procédé de production d'une composition pour complément alimentaire comprenant l'étape de mélange du constituant suivant par rapport au poids total de la composition :
- environ 50 à 70 % en poids d'un véhicule contenant une protéine végétale,
- environ 15 à 25 % en poids d'une source végétale de glucide,
- environ 3 à 15 % en poids d'une source végétale de lipide,
- environ 7 à 14 % en poids de sel, et
- environ 1 à 6 % en poids d'un principe actif
en vue de former un mélange sensiblement homogène, dans lequel le principe actif est ajouté sous forme d'un prémélange sensiblement homogène constitué de ce qui suit, par rapport au poids total dudit principe actif :
- 14 à 26 % en poids de *zingiber offizinalis,*
- 14 à 26 % en poids de *piper longum,*
- 14 à 26 % en poids de *piper nigrum,*
- 14 à 26 % en poids de *cucumis trigonus,* et
- 14 à 26 % en poids de *glycyrrhiza glabra.*

13. Procédé selon la revendication 12, dans laquelle le véhicule contenant la protéine végétale est choisi parmi l'avoine, l'orge ou le maïs, de préférence le véhicule contenant la protéine végétale choisie est l'avoine.

14. Procédé selon la revendication 12 ou 13, dans laquelle la source végétale de glucide est choisie parmi la caroube, la betterave sucrière ou la canne à sucre, de préférence la source végétale de glucide choisie est la caroube.

15. Procédé selon la revendication 12, 13 ou 14, dans laquelle la source végétale de lipide est choisie parmi les noix, les graines de tournesol, l'huile de colza, de préférence la source végétale de lipide est choisie parmi les noix, de manière plus particulièrement préférée la source végétale de lipide est choisie parmi les noisettes.

16. Procédé selon les revendications 12 à 15, dans laquelle la composition de mélange comprend en outre un édulcorant et/ou un arôme à raison d'environ 0,1 à 1,5 % en poids par rapport au poids total de la composition.

17. Procédé selon l'une quelconque des revendications 12 à 16, comprenant l'étape supplémentaire de granulation de la composition pour complément alimentaire produite en granulés, de préférence en granulés ayant des diamètres dans la gamme allant de 5 à 25 mm.

18. Utilisation de la composition pour complément alimentaire selon les revendications 1 à 11 en vue d'améliorer la fertilité chez les mammifères.

19. Utilisation selon la revendication 18, dans laquelle les mammifères sont des animaux d'élevage, de préférence les porcins, les chevaux et les bovins.

20. Utilisation de la composition pour complément alimentaire selon les revendications 1 à 11 en vue de la fabrication d'une composition medicale ou alimentaire pour améliorer la résistance aux maladies provoquées par des bactéries ou des virus chez les mammifères.
